# FASCICULE DE BREVET EUROPEEN

(11) **EP 0 702 549 B1**
(45) Date de publication et mention de la délivrance du brevet: **23.04.1997**
(21) Numéro de dépôt: 94917693.7
(22) Date de dépôt: 31.05.1994
(51) Int. Cl.: A61K 7/50, A61K 7/06, A61K 7/48

(54) **COMPOSITIONS COSMETIQUES CONTENANT AU MOINS UN TENSIO-ACTIF ANIONIQUE DU TYPE ALKYLGALACTOSIDE URONATE ET AU MOINS UNE HUILE HYDROCARBONEE SYNTHETIQUE**
KOSMETISCHE MITTEL ENTHALTENDE MINDESTENS EINEN ANIONISCHEN TENSIDE VOM TYP ALKYLLGALAKTOSIDE-URONAT UND MINDESTENS EIN SYNTHETISCHES KOHLENWASSERSTOFFÖL
COSMETIC COMPOUNDS CONTAINING AT LEAST ONE ANIONIC ALKYLGALACTOSIDE URONATETYPE SURFACE-ACTIVE AGENT AND AT LEAST ONE SYNTHETIC HYDROCARBON OIL

(30) Priorité: 01.06.1993 FR 9306532
(43) Date de publication de la demande: 27.03.1996
(73) Titulaire: L'OREAL, 75008 Paris (FR)
(72) Inventeur: CAUWET, Danièle, F-75011 Paris (FR); DUBIEF, Claude, F-78150 Le Chesnay (FR)
(74) Mandataire: Casalonga, Axel
(86) Numéro de dépôt international: FR9400632
(87) Numéro de publication internationale: WO9427574

(56) Documents cités:
- EP-A- 0 532 370
- EP-A- 0 550 276

## Description

L'invention concerne des compositions cosmétiques contenant au moins un tensio-actif anionique du type alkylgalactoside uronate et une huile hydrocarbonée synthétique ainsi que leur utilisation pour le traitement des matières kératiniques comme revendiquées.

Les compositions de lavage des cheveux ou de la peau sont généralement formulées à partir d'agents tensio-actifs anioniques ou non-ioniques ou leurs mélanges en présence éventuellement d'agents tensio-actifs amphotères.

Les compositions de lavage des cheveux contenant uniquement ces tensio-actifs ne conduisent pas à de bonnes propriétés cosmétiques, en particulier le démêlage des cheveux mouillés est difficile, le volume et la qualité des mousses sont insuffisants.

On a proposé de rajouter à ces compositions de lavage des huiles naturelles ou synthétiques pour améliorer les propriétés cosmétiques. Cependant, le pouvoir moussant de telles compositions et la qualité des mousses restent insuffisants.

Les tensio-actifs anioniques du type alkylgalactoside uronate ont déjà été préconisés dans des compositions lavantes pour les cheveux. Ils ont été décrits dans la demande de brevet EP 0 532 370.

La demanderesse vient de découvrir de manière surprenante, que l'association, dans des compositions lavantes et/ou traitantes pour matières kératiniques, d'un tensio-actif anionique du type alkylgalactoside uronate et d'une huile hydrocarbonée synthétique conférait à ces compositions des propriétés de démêlage des cheveux mouillés améliorées.

Par ailleurs, l'association conforme à la présente invention permet d'obtenir une mousse abondante, compacte et très douce.

En outre, la demanderesse a constaté que les compositions cosmétiques contenant une telle association confèrent de bonnes propriétés cosmétiques telles que la douceur, un toucher agréable.

La présente invention a donc pour objet des compositions cosmétiques contenant au moins un agent tensio-actif anionique du type alkylgalactoside uronate et une huile hydrocarbonée synthétique.

Un autre objet de l'invention est constitué par l'utilisation de ces compositions pour le traitement et/ou le lavage des matières kératiniques telles que les cheveux ou la peau.

Un autre objet concerne un procédé de traitement cosmétique des cheveux ou de la peau au moyen des compositions de l'invention; les procédés de lavage et de traitement des cheveux étant préférés.

Les compositions cosmétiques selon l'invention contiennent dans un milieu aqueux cosmétiquement acceptable :
(**A**) au moins un agent tensio-actif anionique du type alkylgalactoside uronate de formule : 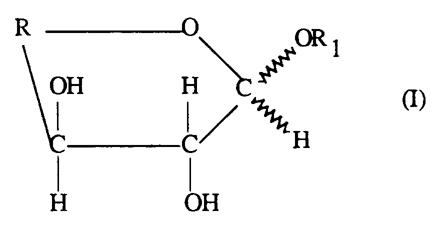 dans laquelle :
   R₁ désigne un radical alkyle linéaire ou ramifié de 8 à 22 atomes de carbone.
   R désigne un groupe
   (i) 〉CH -CH(OH) - CO₂R₂ ou
   (ii) 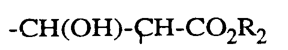 dont le carbone portant le groupe hydroxyle est relié à l'atome d'oxygène endocyclique; R₂ étant un hydrogène, un métal alcalin, un métal alcalino-terreux ou un groupe ammonium quaternaire non substitué ou substitué par des radicaux alkyle, hydroxyalkyle ou un groupe ammonium dérivé d'amino-acides.
(**B**) et au moins une huile hydrocarbonée synthétique choisie parmi les poly-α-oléfines.

Les tensio-actifs anioniques du type alkylgalactoside uronate de formule (I) sont connus et peuvent être préparés selon les procédés décrits dans la demande de brevet EP-A-O 532 370.

Le métal alcalin est notamment le sodium, le potassium et le métal alcalino-terreux est de préférence le magnésium. Comme sels d'ammonium quaternaires, on peut citer les sels d'ammoniaque, de triéthanolamine, de monoéthanolamine, de 2-amino 2-méthyl 1,3-propanediol de 2-méthyl 2-amino 1-propanol; l'amino acide est notamment l'histidine, l'arginine ou la lysine.

On utilise de préférence les composés de formule (I) pour lesquels le radical R₁ désigne un alkyle en C₈-C₁₄ et plus particulièrement le radical décyle.

On utilise notamment les composés suivants :
Décyl α-D-galactopyranoside uronate de sodium : 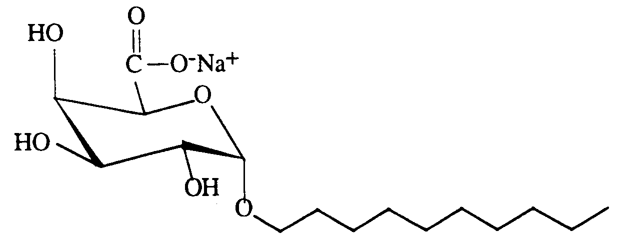
Décyl β-D-galactopyranoside uronate de sodium : 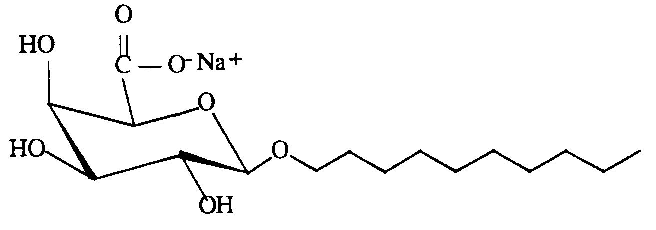
Décyl α-D-galactofuranoside uronate de sodium : 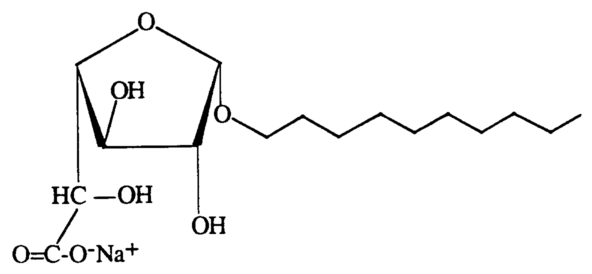
Décyl β-D-galactofuranoside uronate de sodium :

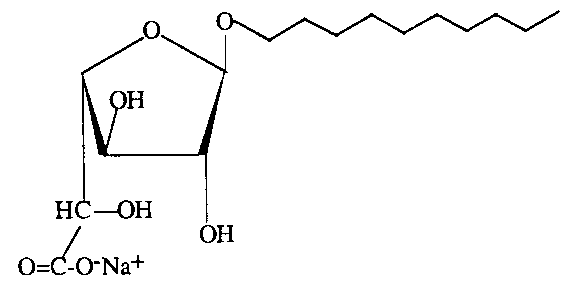

Les huiles hydrocarbonées synthétiques sont des poly-α-oléfines et en particulier :
a) de type polybutène hydrogéné ou non et de préférence polyisbutène hydrogéné ou non;
   on utilise de préférence les oligomères d'isobutylène de poids moléculaire inférieur à 1000 et leurs mélanges avec des polyisobutylènes de poids moléculaire supérieur à 1000 et de préférence compris entre 1000 et 15000; ces produits sont vendus par exemple par la société AMOCO sous la dénomination INDOPOL, par la société PRESPERSE sous les dénominations PERMETHYL 99A, 101A, 102A, 104A, 106A ou par la société ICI sous la dénomination ARLAMOL HD;
b) de type polydécène hydrogéné ou non;
   de tels produits sont vendus par exemple par la société ETHYL CORPORATION sous les dénominations ETHYLFLO, par la société ICI sous les dénominations ARLAMOL PAO.

Les alkylgalactoside uronates de formule (I) sont utilisés dans les compositions conformes à l'invention dans des proportions comprises de préférence entre 0,5 et 30% en poids par rapport au poids total de la composition.

Les huiles hydrocarbonées sont utilisées dans les compositions de l'invention dans des proportions comprises de préférence entre 0,1 et 20% en poids par rapport au poids total de la composition.

Les compositions conformes à l'invention peuvent contenir en plus un agent épaississant et/ou un agent de mise en suspension de l'huile tels que des alcalnolamides d'acides gras, des acides polyacryliques, des dérivés de cellulose, des esters d'acides gras et de polyéthylèneglycol les copolymères réticulés d'acrylamide et d'un monomère choisi parmi l'acrylate d'ammonium, l'acide 2-acrylamido 2-méthylpropane sulfonique partiellement ou totalement neutralisé ou le chlorure de méthacryloyloxyéthyl triméthylammonium; les uréthannes polyéthers, les copolymères de méthylvinyléther/acide maléïque réticulés.

On peut également citer comme agent de mise en suspension de l'huile les composés choisis parmi
a) ceux de formule :

   R₃X (II)

   dans laquelle R₃ est un radical aliphatique à longue chaîne carbonée, éventuellement interrompue par un ou plusieurs atomes d'oxygène, et X est un reste acide carboxylique, sulfurique ou phosphorique ou un radical dérivé d'un acide carboxylique ou d'un amide; ces composés de formule (II) sont choisis parmi ceux dans lesquels :
   (i) R₃ est un radical alkyle ou alcényle en C₁₁-C₂₁;
      et X est
      - un groupement COOA où A est un radical mono- ou polyhydroxyalkyle dérivé d'un polyol en C₂-C₃ ou un radical CH₂CH₂SO₃M;
      - un groupement CO(OCH₂CH₂)ₖOH où k a une valeur comprise entre 2 et 150;
      - un groupement 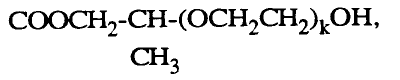 où k a une valeur comprise entre 2 et 150, les fonctions OH libres des groupements définis ci-dessus pouvant être estérifiées par un acide R'COOH où R' est un alkyle ou alcényle en C₁₁-C₂₁;
      - un groupement CONR₄R₅ où R₄ et R₅ représentent hydrogène ou hydroxyalkyle en C₁-C₄, l'un au moins représentant hydroxyalkyle en C₁-C₄;
      - un groupement OSO₃M ou 1/3 PO₄ ³⁻ M₃ où M représente un métal alcalin, ammonium ou un reste d'alcanolamine en C₁-C₄;
   (ii) R₃ désigne un radical R₆O(C₂H₄O)ₗ CH₂ et X désigne un groupement COOM où M a la signification indiquée ci-dessus, R₆ désignant un radical alkyle en C₁₂-C₁₄ et l un nombre entier ou décimal compris entre 2,5 et 10, ou bien R₆ désigne oléyle et l varie de 2 à 9 ou encore R₆ désigne alkyle en (C₈-C₉) phényle et l varie de 4 à 8 , ou les dérivés dans lesquels R₆ désigne un groupement alkyle(C₁₂-C₁₆) et X un groupement CONR₄R₅; dans lequel R₄ et R₅ ont la même signification que celle indiquée ci-dessus et l a une valeur de 1 à 3 inclus;
b) des oxydes d'amines de formule : 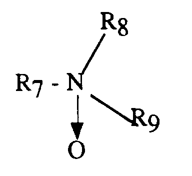 dans laquelle R₇ désigne un groupement alkyle en C₁₆-C₂₂, et R₈ et R₉, identiques ou différents, représentent un groupement alkyle en C₁-C₄ ou hydroxyalkyle en C₁-C₄;
c) les biopolysaccharides choisis parmi les gommes de xanthane et les scléroglucanes.

Si les compositions selon l'invention ne sont pas utilisées pour le lavage des matières kératiniques, la concentration en tensio-actifs anioniques de formule (I) est comprise entre 1 et 10% et plus particulièrement entre 1 et 5% en poids par rapport au poids total de la composition. Ces compositions sont utilisées notamment comme compositions à rincer ou non, appliquées avant ou après shampooing, une coloration, une décoloration, une permanente, un défrisage ou dans des compositions de décoloration, de coloration, de permanente ou de défrisage.

Lorsque les compositions selon l'invention sont des compositions lavantes, elles contiennent les agents tensio-actifs de formule (I) dans une concentration comprise entre 4 et 50% en poids et de préférence entre 8 et 40% en poids par rapport au poids total de la composition.

Les compositions peuvent contenir en plus des agents tensio-actifs supplémentaires de nature anionique, non-ionique, amphotère, zwitterionique ou cationique.

Parmi les agents tensio-actifs anioniques, on peut citer les sels alcalins, les sels d'ammonium, les sels d'amines, les sels d'aminoalcools, les sels de magnésium des composés suivants : les acides gras, les alkylsulfates, les alkyléthersulfates, les alkylamidoéthersulfates, les alkylarylpolyéthersulfates, les monoglycérides sulfates; les alkyléthersulfonates, les alkylsulfonates, les alkylamides sulfonates, les alkylarylsulfonates, les oléfines sulfonates, les parraffines sulfonates; les alkylsulfosuccinates, les alkyléthersulfosuccinates, les alkylamides sulfosuccinates; les alkylsulfosuccinamates; les acylglutamates; les alkylsulfoacétates; les alkylétherphosphates; les acylsarcosinates; les N-acyltaurates; les iséthionates.

Le radical alkyle ou acycle de ces différents composés est généralement constitué par une chaîne carbonée comportant de 10 à 20 atomes de carbone.

On peut également utiliser des agents tensio-actifs faiblement anioniques, tels que les acides alkylamides ou alkyléthers carboxyliques polyoxyalkylénés, tels que ceux comportant 2 à 50 groupements oxyde d'éthylène.

Les agents tensio-actifs non-ioniques sont plus particulièrement choisis parmi les alcools, les α-diols ou les alkylphénols ou les acides gras polyéthoxylés ou polypropoxylés à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut citer plus particulièrement les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amines grasses polyéthoxylées ayant de préférence 2 à 30 moles d'oxyde d'éthylène; les esters d'acides gras de sorbitan oxyéthylénés ayant de préférence 2 à 30 moles d'oxyde d'éthylène; les esters d'acides gras de sucre, les esters d'acides gras du polyéthylèneglycol, les esters d'acides gras de glycols; les oxydes d'amines tels que les oxydes d'alkyl(C₁₀-C₁₄) amines ou de N-acylamidopropylmorpholine.

Les agents tensio-actifs amphotères ou zwittérioniques préférés sont les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆)bétaïnes ou les alkyl(C₈-C₂₀)amidoalkyl(C₁-C₆)sulfobétaïnes.

On peut également citer les alkylpeptides, les alkylimidazolium bétaïnes.

Parmi les dérivés d'amines, on peut citer les produits commercialisés sous la dénomination MIRANOL, tels que ceux décrits dans les brevets US-A-2 528 378 et 2 781 354 ou classés dans le dictionnnaire CTFA, 3ème édition, 1982, sous les dénominations d'Amphocarboxyglycinates ou d'Amphocarboxypropionates.

Les agents tensio-actifs cationiques sont choisis parmi les sels d'ammonium quaternaires tels que les halogénures d'alkyl(C₈-C₂₂) triméthyl ammonium, les halogénures de dialkyl(C₈-C₂₂)diméthyl ammonium, les halogénures d'alkyl(C₈-C₂₂)diméthylhydroxyéthylammonium.

Les co-tensio-actifs additionnels peuvent représenter jusqu'à 50% du poids total des tensio-actifs présents dans la composition.

Le pH des compositions conforme à l'invention est généralement compris entre 2 et 10,5 plus particulièrement entre 3 et 8.

Dans la mesure où le milieu cosmétiquement acceptable de la composition selon l'invention est un milieu aqueux, il peut être constitué uniquement par de l'eau ou par un mélange d'eau et d'un solvant cosmétiquement acceptable, tel que des alcools inférieurs en C₁-C₄ comme l'éthanol, l'isopropanol, le n-butanol; les alkylèneglycols comme le propylèneglycol, les éthers de glycols.

Les compositions selon l'invention peuvent se présenter sous forme de liquides plus ou moins épaissis, de gels, d'émulsions (laits ou crèmes), de lotions hydroalcooliques, de dispersions, de mousses aérosol ou de pains solides.

Les compositions sont par exemple des lotions, des laits ou des crèmes émollients, des lotions, des laits ou des crèmes pour les soins des matières kératiniques, des crèmes ou des laits démaquillants, des bases de fonds de teint, des lotions, des laits ou des crèmes antisolaires, des lotions, des laits ou des crèmes de bronzage artificiel, des crèmes ou des mousses de rasage, des lotions après rasage, des masques pour le visage, des produits de maquillage pour les yeux, des fards et fonds de teint pour le visage, des vernis à ongles, des shampooings, des produits pour le bain ou la douche, des compositions à rincer ou non, à appliquer avant ou après un shampooing, une coloration, une décoloration, une permanente ou un défrisage, des compositions de coloration, de décoloration, de permanente ou de défrisage des cheveux.

Les compositions conformes à l'invention peuvent également contenir en plus divers additifs tels que des renforçateurs de mousse, des séquestrants, des parfums, des électrolytes, des corps gras tels que des alcools gras, des céramides, des huiles minérales, végétales, animales, des cires minérales, végétales, animales ou synthétiques, des filtres UV, des agents anti-radicaux libres, des agents nacrants, des biocides, des antibactériens, des agents antipelliculaires, des agents anti-séborrhéïques, des anti-parasitaires, des repellents, des colorants, des pigments, des oxydants, des réducteurs, des hydratants, des polymères anioniques et non ioniques, des vitamines, des α-hydroxyacides.

Le procédé de lavage et/ou de conditionnement des matières kératiniques et en particulier des cheveux ou de la peau conforme à l'invention, consiste à appliquer sur ces matières au moins une composition telle que définie ci-dessus, cette application étant suivie éventuellement d'une étape de rinçage à l'eau.

Les compositions de lavage peuvent être utilisées comme shampooing mais également comme gel-douche pour le lavage des cheveux et de la peau, auquel cas ils sont appliqués sur la peau et les cheveux humides qui sont rincés après application.

Lorsque les compositions sont utilisées pour le conditionnement des cheveux, elles sont appliquées sur les cheveux humides, après quoi on peut soit les sécher soit après un temps de pose de 1 à 10 minutes, les rincer à l'eau. On constate que les cheveux humides se démêlent bien.

Les exemples qui suivent servent à illustrer l'invention sans toutefois présenter un caractère limitatif.

### EXEMPLE 1

### SHAMPOOING

| | |
|---|---|
| - Décyl - D-galactoside uronate de sodium | 10 g M |
| - Lauryl éther sulfate de sodium (C₁₂/C₁₄ 70/30) à 2.2 OE en solution aqueuse à 28 % vendu sous le nom d'Empicol ESB / 3 FL par MARCHON | 10 g MA |
| - Iso-eicosane vendu sous le nom de Perméthyl 10 2 A par CREATIONS COULEURS | 2 g |
| - Gomme hydroxy propylguar vendu sous le nom de Jaguar HP 60 par MEYHALL | 0,75 g MA |
| Colorants, parfum, conservateur | |
| eau qsp | 100 g |
| pH ajusté à 7 par NaOH | |

Aspect : opalescent et visqueux

### EXEMPLE 2

### APRES SHAMPOOING

| | |
|---|---|
| - Décyl - D-galactoside uronate de sodium | 4 g |
| - Heptaméthylnonane vendu sous le nom d'Arlamol HD par ICI | 10 g |
| - Acide polyacrylique réticulé vendu sous le nom de Carbopol 980 par la Société GOODRICH | 1,6 g MA |
| Colorants, parfum, conservateur | |
| eau qsp | 100 g |
| pH ajusté à 5 par NaOH | |

La composition se présente sous la forme d'une crème blanche et visqueuse.

### EXEMPLE 3

### BAIN MOUSSANT

| | |
|---|---|
| - Décyl - D-galactoside uronate de sodium | 20 g MA |
| - N-cocoamido éthyl N-éthoxycarboxy méthyl glycinate de sodium | 10 g MA |
| - Heptaméthylnonane vendu sous le nom d'Arlamol HD par ICI | 5 g |
| - Diuréthane d'alcools (C₁₆/C₁₈) oxyéthylèné et oxypropylèné vendu sous le nom de Dapral T 212 par la Société AKZO | 2,5 g |
| Colorants, parfum, conservateur | |
| eau qsp | 100 g |
| pH ajusté à 8 par NaOH | |

La composition se présente sous la forme d'un liquide opalescent, légèrement visqueux.

## Revendications

1. Composition cosmétique, caractérisée par le fait qu'elle contient dans un milieu aqueux cosmétiquement acceptable :
(**A**) au moins un agent tensio-actif anionique du type alkylgalactoside uronate de formule : 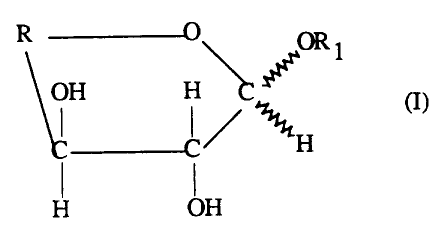 dans laquelle :
R₁ désigne un radical alkyle linéaire ou ramifié en C₈-C₂₂
R désigne un groupe
(i) 〉CH -CH(OH) - CO₂R₂ ou
(ii) -CH(OH)-CH-CO₂R₂ dont le carbone portant le groupe hydroxyle est relié à l'atome d'oxygène endocyclique; R₂ désigne un hydrogène, un métal alcalin ou un métal alcalino terteux ou un groupement ammonium quaternaire non substitué ou substitué par des alkyles, hydroxyalkyles ou un groupement ammonium dérivé d'amino-acides;
(**B**) au moins une huile hydrocarbonée synthétique choisie parmi les poly-α-oléfines.

2. Composition selon la revendication 1, caractérisée par le fait que dans la formule (I), le radical R₂ désigne le sodium ou le potassium; le magnésium; le groupe ammonium quaternaire dérivé d'ammoniaque, de triéthanolamine, de monoéthanolamine, de 2-amino 2-méthyle 1,3-propanediol de 2-méthyl 2-amino 1-propanol, d'histidine, d'arginine ou de lysine.

3. Composition selon la revendication 1 ou 2, caractérisé par le fait que le composé de formule (I) est choisi parmi ceux pour lesquels R₁ désigne un alkyle en C₈-C₁₄.

4. Composition selon l'une quelconque des revendications 1 à 3, caractérisée par le fait que les composés de formule (I) sont choisis parmi ceux pour lesquels R₁ désigne le radical décyle.

5. Composition selon la revendication 4, caractérisée par le fait que le composé de formule (I) est choisi parmi :
le décyl α-D-galactopyranoside uronate de sodium
le décyl β-D-galactopyranoside uronate de sodium
le décyl α-D-galactofuranoside uronate de sodium
le décyl β-D-galactofuranoside uronate de sodium.

6. Composition selon l'une quelconque des revendications 1 à 5, caractérisée par le fait que les poly α-oléfines sont du type polydécène hydrogéné ou non ou polybutène hydrogéné ou non.

7. Composition selon l'une quelconque des revendications 1 à 6, caractérisée par le fait que les agents tensio-actifs anioniques de formule (I) sont présents dans des proportions comprises entre 0,5 et 30% en poids et les huiles hydrocarbonées synthétiques sont présentes dans des proportions comprises entre 0,1 et 20% en poids; les pourcentages en poids étant exprimés par rapport au poids total de la composition.

8. Composition de conditionnement des matières kératiniques selon l'une quelconque des revendications 1 à 7, caractérisée par le fait que la concentration en agents tensio-actifs anioniques de formule (I) est comprise entre 1 et 10% en poids par rapport au poids total de la composition.

9. Composition de lavage des matières kératiniques selon l'une quelconque des revendications 1 à 7, caractérisée par le fait que la concentration en tensio-actifs anioniques de formule (I) est comprise entre 4 et 50% en poids par rapport au poids total de la composition.

10. Composition selon l'une quelconque des revendications 1 à 9, caractérisée par le fait qu'elle contient en plus un co-tensio-actif additionnel du type anionique, non-ionique, amphotère ou cationique dans une proportion allant jusqu'à 50% du poids total en agents tensio-actifs.

11. Composition de lavage selon la revendication 10, caractérisée par le fait que le co-tensio-actif anionique additionnel est choisi parmi les sels alcalins, les sels d'ammonium, les sels d'amines, les diaminoalcools ou les sels de magnésium des composés : acides gras; alkylsulfates, alkyléthersulfates, alkylamidoéthersulfates, alkylarylpolyéthersulfates, monoglycérides sulfates, alkylamides sulfates; alkylsulfonates, alkyléthersulfonates, alkylsulfosuccinates, alkyléthersulfosuccinates, alkylamides sulfosuccinates; acylglutamates; alkylsulfosuccinamates; alkylsulfoacétates; alkylétherphosphates; acylsarcosinates; N-acyltaurates; isethionates; le radical alkyle ou acyle étant constitué d'une chaîne carbonée compôrtant de 10 à 20 atomes de carbone ou bien des acides alkylamides ou alkyléthers carboxyliques polyoxyalkylénés.

12. Composition de lavage selon la revendication 10, caractérisée par le fait que le co-tensio-actrif non-ionique est choisi parmi les alcools, les α-diols ou les alkylphénols; les acides gras polyéthoxylés ou polypropoxylés à chaine grasse comportant 8 à 18 atomes de carbone, le nombre de groupes oxyde d'éthylène ou oxyde de propylène étant compris entre 2 et 50 et le nombre de groupes glycérol étant compris entre 2 et 30; les copolymères d'oxydes d'éthylène et de propylène; les condensats d'oxydes d'éthylène et de propylène sur des alcools gras; les amides gras polyéthoxylés; les amines grasses polyéthoxylées; les esters d'acides gras de sucre; les esters d'acides gras du polyéthylèneglycol; les esters d'acides gras de glycols; les oxydes d'amines.

13. Composition selon la revendication 10, caractérisée par le fait que le co-tensio-actif amphotère ou zwitterionique additionnel est choisi parmi les dérivés d'amines secondaires ou tertiaires aliphatiques dans lesquels le radical aliphatique est une chaine linéaire ou ramifiée comportant 8 à 18 atomes de carbone et qui contient au moins un groupe anionique hydrosolubilisant carboxylate, sulfonate, sulfate, phosphate ou phosphonate; les alkyl (C₈-C₂₀) bétaïnes; les sulfobétaïnes, les alkyl (C₈-C₂₀)amidoalkyl (C₁-C₆)bétaïnes ou les alkyl(C₈-C₂₀)amidoalkyl(C₁-C₆) sulfobétaïnes; les alkylpeptides; les alkylimidazoliumbétaïnes.

14. Composition de lavage selon la revendication 10, caractérisée par le fait que le co-tensio-actif cationique additionnel est choisi parmi les sels d'ammonium quaternaire.

15. Composition selon l'une quelconque des revendications 1 à 14, caractérisée par le fait qu'elle contient en plus un agent épaisissant et/ou un agent de mise en suspension de l'huile hydrocarbonée synthétique.

16. Composition selon la revendication 15, caractérisée par le fait que l'agent épaississant et/ou l'agent de suspension est choisi parmi les alcanolamides d'acides gras; les acides polyacryliques; les dérivés de cellulose; les esters d'acides gras et de polyéthylèneglycol; les copolymères réticulés d'acrylamide et d'un monomère choisi parmi l'acrylate d'ammonium, l'acide 2-acrylamido 2-méthylpropane sulfonique ou le chlorure de méthacryloyloxyéthyl triméthylammonium; les uréthanes polyéthers; les copolymères de méthylvinyléther/acide maléïque réticulés.

17. Composition selon la revendication 15, caractérisée par le fait que l'agent de suspension de l'huile hydrocarbonée synthétique est choisi parmi les composés suivants
a) ceux de formule :
R₃X (II)
où R₃ est un radical aliphatique à longue chaîne carbonée, éventuellement interrompue par un ou plusieurs atomes d'oxygène et X est un reste d'acide carboxylique, sulfurique ou phosphorique ou un radical dérivé d'un acide carboxylique ou d'un amide;
b) des oxydes d'amines de formule 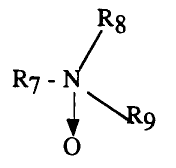 dans laquelle R₇ désigne un alkyle en C₁₆-C₂₂ et R₈ et R₉, identiques ou différents représentent un alkyle en C₁-C₄ ou hydroxyalkyle en C₁-C₄;
c) les biopolysaccharides.

18. Composition selon l'une quelconque des revendications 1 à 17, caractérisée par le fait que le milieu cosmétiquement acceptable est constitué par de l'eau ou un mélange d'eau et d'un solvant cosmétiquement acceptable.

19. Composition selon l'une quelconque des revendications 1 à 18, caractérisée par le fait qu'elle se présente sous forme de liquide plus ou moins épaissi, de gel, d'émulsion, de lotion hydroalcoolique, de dispersion, de pain solide ou de mousse aérosol.

20. Composition selon l'une quelconque des revendications 1 à 19, caractérisée par le fait qu'elle contient en plus des additifs choisis parmi les renforçateurs de mousse, les sequestrants, les électrolytes, parfums, les conservateurs, les alcools gras, les huiles minérales, végétales ou animales, les cires minérales, végétales, animales ou synthétiques, les céramides, les filtres UV, les agents nacrants, les anti-radicaux libres, les biocides, les antibactériens, les agents antipelliculaires, antiséborrhéïques, anti-parasitaires, les repellents, des colorants, des pigments, des oxydants, des réducteurs, des hydratants, des polymères anioniques ou non-ioniques, des vitamines ou des α-hydroxyacides.

21. Utilisation de la composition telle que définie dans l'une quelconque des revendications 1 à 20 pour le traitement et/ou le lavage des matières kératiniques constituées par les cheveux ou la peau.

22. Procédé de lavage et/ou de conditionnement cosmétique des cheveux ou de la peau, caractérisé par le fait qu'il consiste à appliquer sur la peau ou les cheveux une quantité efficace de composition selon l'une quelconque des revendications 1 à 20, cette application étant éventuellement suivie d'un rinçage à l'eau.

## Claims

1. Cosmetic composition, characterized in that it contains, in a cosmetically acceptable aqueous medium:
**(A)** at least one anionic surfactant of alkylgalactoside uronate type of formula: 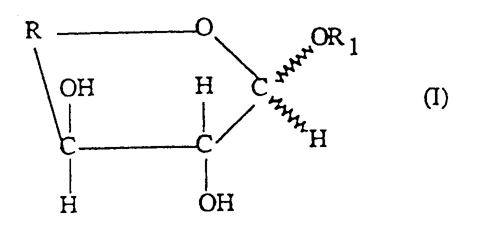 in which:
R₁ denotes a linear or branched C₈-C₂₂ alkyl radical
R denotes a group
(i) 〉CH-CH(OH) - CO₂R₂ or
(ii) -CH(OH)-CH-CO₂R₂, in which the carbon carrying the hydroxyl group is connected to the endocyclic oxygen atom; R₂ denotes a hydrogen, an alkali metal or an alkaline-earth metal or a quaternary ammonium group which is unsubstituted or substituted by alkyls or hydroxyalkyls or an ammonium group derived from amino acids;
**(B)** at least one synthetic hydrocarbon oil chosen from the poly-α-olefins.

2. Composition according to Claim 1, characterized in that, in the formula (I), the radical R₂ denotes sodium or potassium; magnesium; or the quaternary ammonium group derived from ammonia, triethanolamine, monoethanolamine, 2-amino-2-methyl-1,3-propanediol, 2-methyl-2-amino-1-propanol, histidine, arginine or lysine.

3. Composition according to Claim 1 or 2, characterized in that the compound of formula (I) is chosen from those in which R₁ denotes a C₈-C₁₄ alkyl.

4. Composition according to any one of Claims 1 to 3, characterized in that the compounds of formula (I) are chosen from those in which R₁ denotes the decyl radical.

5. Composition according to Claim 4, characterized in that the compound of formula (I) is chosen from:
sodium decyl α-D-galactopyranoside uronate
sodium decyl β-D-galactopyranoside uronate
sodium decyl α-D-galactofuranoside uronate
sodium decyl β-D-galactofuranoside uronate.

6. Composition according to any one of Claims 1 to 5, characterized in that the poly-α-olefins are of hydrogenated or nonhydrogenated polydecene or hydrogenated or nonhydrogenated polybutene type.

7. Composition according to any one of Claims 1 to 6, characterized in that the anionic surfactants of formula (I) are present in proportions of between 0.5 and 30% by weight and the synthetic hydrocarbon oils are present in proportions of between 0.1 and 20% by weight; the percentages by weight being expressed with respect to the total weight of the composition.

8. Composition for conditioning keratinous substances according to any one of Claims 1 to 7, characterized in that the concentration of anionic surfactants of formula (I) is between 1 and 10% by weight with respect to the total weight of the composition.

9. Composition for washing keratinous substances according to any one of Claims 1 to 7, characterized in that the concentration of anionic surfactants of formula (I) is between 4 and 50% by weight with respect to the total weight of the composition.

10. Composition according to any one of Claims 1 to 9, characterized in that it furthermore contains an additional cosurfactant of anionic, nonionic, amphoteric or cationic type in a proportion ranging up to 50% of the total weight of surfactants.

11. Washing composition according to Claim 10, characterized in that the additional anionic cosurfactant is chosen from the alkali metal salts, the ammonium salts, the amine salts, the diaminoalcohols [sic] or the magnesium salts of the compounds: fatty acids; alkyl sulfates, alkyl ether sulfates, alkylamidoether sulfates, alkylarylpolyether sulfates, monoglyceride sulfates or alkylamide sulfates; alkylsulfonates, alkylethersulfonates, alkylsulfosuccinates, alkylethersulfosuccinates or alkylamidesulfosuccinates; acylglutamates; alkylsulfosuccinamates; alyylsulfoacetates; alkyl ether phosphates; acylsarcosinates; N-acyltaurates; or isethionates; the alkyl or acyl radical consisting of a carbon chain containing from 10 to 20 carbon atoms or else polyoxyalkylenated alkyl amide or alkyl ether carboxylic acids.

12. Washing composition according to Claim 10, characterized in that the nonionic cosurfactant is chosen from the alcohols, the α-diols or the alkylphenols; the polyethoxylated or polypropoxylated fatty acids [sic], with a fatty chain containing 8 to 18 carbon atoms, the number of ethylene oxide or propylene oxide groups being between 2 and 50 and the number of glycerol groups being between 2 and 30 [sic]; the copolymers of ethylene oxide and of propylene oxide; the condensates of ethylene oxide and of propylene oxide with fatty alcohols; the polyethoxylated fatty amides; the polyethoxylated fatty amines; the fatty acid esters of sugar [sic]; the fatty acid esters of polyethylene glycol; the fatty acid esters of glycols; or the amine oxides.

13. Composition according to Claim 10, characterized in that the additional amphoteric or zwitterionic cosurfactant is chosen from the derivatives of secondary or tertiary aliphatic amines, in which the aliphatic radical is a linear or branched chain containing 8 to 18 carbon atoms and which contains at least one water-solubilizing carboxylate, sulfonate, sulfate, phosphate or phosphonate anionic group; the (C₈-C₂₀)alkylbetaines; the sulfobetaines, the (C₈-C₂₀)alkylamido(C₁-C₆)alkylbetaines or the (C₈-C₂₀)alkylamido(C₁-C₆)alkylsulfobetaines; the alkylpeptides; or the alkylimidazoliumbetaines [sic].

14. Washing composition according to Claim 10, characterized in that the additional cationic cosurfactant is chosen from the guaternary ammonium salts.

15. Composition according to any one of Claims 1 to 14, characterized in that it furthermore contains a thickening agent and/or a suspending agent for the synthetic hydrocarbon oil.

16. Composition according to Claim 15, characterized in that the thickening agent and/or the suspending agent is chosen from fatty acid alkanolamides; poly(acrylic acid)s; cellulose derivatives; esters of fatty acids and of polyethylene glycol; crosslinked copolymers of acrylamide and of a monomer chosen from ammonium acrylate, 2-acrylamido-2-methylpropanesulfonic acid or methacryloyloxyethyltrimethylammonium chloride; polyetherurethanes; or crosslinked methyl vinyl ether-maleic acid copolymers.

17. Composition according to Claim 15, characterized in that the suspending agent for the synthetic hydrocarbon oil is chosen from the following compounds
a) those of formula:
R₃X (II)
where R₃ is an aliphatic radical with a long carbon chain, optionally interrupted by one or a number of oxygen atoms, and X is a carboxylic, sulfuric or phosphoric acid residue or a radical derived from a carboxylic acid or from an amide;
b) amine oxides of formula 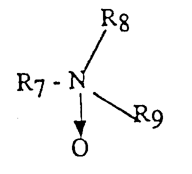 in which R₇ denotes a C₁₆-C₂₂ alkyl and R₈ and R₉, which are identical or different, represent a C₁-C₄ alkyl or C₁-C₄ hydroxyalkyl;
c) biopolysaccharides.

18. Composition according to any one of Claims 1 to 17, characterized in that the cosmetically acceptable medium consists of water or a mixture of water and of a cosmetically acceptable solvent.

19. Composition according to any one of Claims 1 to 18, characterized in that it is provided in the form of a more or less thickened liquid, a gel, an emulsion, an agueous/alcoholic lotion, a dispersion, a solid bar or an aerosol foam.

20. Composition according to any one of Claims 1 to 19, characterized in that it furthermore contains additives chosen from foam reinforcers, sequestering agents, electrolytes, fragrances, preservatives, fatty alcohols, mineral, vegetable or animal oils, mineral, vegetable, animal or synthetic waxes, ceramides, UV screening agents, pearlescence agents, agents for combating free radicals, biocides, antibacterials, antidandruff, anti-seborrheic or antiparasitic agents, repellents, dyes, pigments, oxidizing agents, reducing agents, moisturizers, anionic or nonionic polymers, vitamins or α-hydroxy acids.

21. Use of the composition as defined in any one of Claims 1 to 20 for treating and/or washing keratinous substances consisting of the hair or the skin.

22. Process for cosmetic washing and/or conditioning of the hair or of the skin, characterized in that it consists in applying an effective amount of composition according to any one of Claims 1 to 20 to the skin or to the hair, this application optionally being followed by rinsing with water.

## Patentansprüche

1. Kosmetische Zusammensetzung,
dadurch **gekennzeichnet**, daß sie in einem wässrigen kosmetisch geeigneten Milieu enthält:
(A) mindestens ein anionisches oberflächenaktives Mittel vom Alkylgalactosiduronat-Typ der Formel: 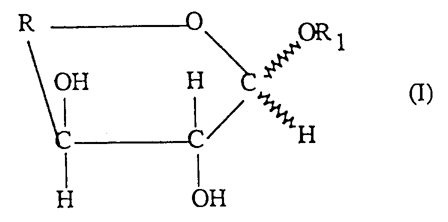 worin gilt:
R₁ bedeutet einen linearen oder verzweigten Alkylrest,
R bedeutet ein Gruppe:
(i) 〉CH-CH(OH)-CO₂R₂ oder
(ii) 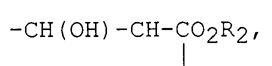 deren die Carboxylgruppe aufweisender Kohlenstoff an das endozyklische Sauerstoffatom gebunden ist, worin R₂ Wasserstoff, ein Alkalimetall, Erdalkalimetall oder eine mit Alkyl- oder Hydroxyalkylresten substituierte oder nicht-substituierte quaternäre Ammoniumgruppe oder eine von Aminosäuren abgeleitete Ammoniumgruppe ist; und
(B) mindestens ein synthetisches Kohlenwasserstofföl, ausgewählt aus Poly-α-olefinen.

2. Zusammensetzung gemäß Anspruch 1,
dadurch **gekennzeichnet**, daß
in der Formel (I) der Rest R₂ Natrium oder Kalium, Magnesium oder eine quaternäre Ammoniumgruppe bedeutet, die von Ammoniak, Triethanolamin, Monoethanolamin, 2-Amino-2-methylpropan-1,3-diol, 2-Methyl-2-aminopropan-1-ol, Histidin, Arginin oder Lysin abgeleitet ist.

3. Zusammensetzung gemäß Anspruch 1 oder 2,
dadurch **gekennzeichnet**, daß
die Verbindung der Formel (I) aus denjenigen ausgewählt ist, für die R₁ einen C₈₋₁₄-Alkylrest bedeutet.

4. Zusammensetzung gemäß einem der Ansprüche 1 bis 3,
dadurch **gekennzeichnet**, daß
die Verbindung der Formel (I) aus denjenigen ausgewählt ist, für die R₁ den Decylrest bedeutet.

5. Zusammensetzung gemäß Anspruch 4,
dadurch **gekennzeichnet**, daß
die Verbindung der Formel (I) ausgewählt ist aus:
Natrium-Decyl-α-D-galactopyranosiduronat,
Natrium-Decyl-β-D-galactopyranosiduronat,
Natrium-Decyl-α-D-galactofuranosiduronat und aus
Natrium-Decyl-β-D-galactofuranosiduronat.

6. Zusammensetzung gemäß einem der Ansprüche 1 bis 5,
dadurch **gekennzeichnet**, daß
die Poly-α-olefine vom gegebenenfalls hydrierten Polydecen- oder Polybuten-Typ sind.

7. Zusammensetzung gemäß einem der Ansprüche 1 bis 6,
dadurch **gekennzeichnet**, daß
die anionischen oberflächenaktiven Mittel der Formel (I) in Mengenanteilen von 0,5 bis 30 Gew.% und die synthetischen Kohlenwasserstofföle in Mengenanteilen von 0,1 bis 20 Gew.% vorhanden sind, wobei die Gewichtsprozent-Angaben auf das Gesamtgewicht der Zusammensetzung bezogen sind.

8. Zusammensetzung gemäß einem der Ansprüche 1 bis 7 zum Konditionieren keratinischer Materien,
dadurch **gekennzeichnet**, daß
die Konzentration an anionischen oberflächenaktiven Mitteln der Formel (I) 1 bis 10 Gew.% beträgt, bezogen auf das Gesamtgewicht der Zusammensetzung.

9. Zusammensetzung gemäß einem der Ansprüche 1 bis 7 zum Waschen keratinischer Materien,
dadurch **gekennzeichnet**, daß
die Konzentration an anionischen oberflächenaktiven Mitteln der Formel (I) 4 bis 50 Gew.% beträgt, bezogen auf das Gesamtgewicht der Zusammensetzung.

10. Zusammensetzung gemäß einem der Ansprüche 1 bis 9,
dadurch **gekennzeichnet**, daß
sie zusätzlich ein weiteres oberflächenaktives Co-Mittel vom anionischen, nicht-ionischen, amphoteren oder kationischen Typ in einem Mengenanteil bis zu 50% des Gesamtgewichts der oberflächenaktiven Mittel enthält.

11. Zusammensetzung gemäß Anspruch 10 zum Waschen,
dadurch **gekennzeichnet**, daß
das anionische zusätzliche oberflächenaktive Co-Mittel ausgewählt ist aus Alkali-, Ammonium-, Amin-, Aminoalkohol- oder Magnesiumsalzen der folgenden Verbindungen: von Fettsäuren, Alkylsulfaten, Alkylethersulfaten, Alkylamidoethersulfaten, Alkylarylpolyethersulfaten, Monoglyceridsulfaten, Alkylamidsulfaten, Alkylsulfonaten, Alkylethersulfonaten, Alkylsulfosuccinaten, Alkylethersulfosuccinaten, Alkylamidsulfosuccinaten, Acylglutamaten, Alkylsulfosuccinamaten, Alkylsulfoacetaten, Alkyletherphosphaten, Acylsarcosinaten, N-Acyltauraten und Isethionaten, wobei der Alkyl- oder Acylrest aus einer Kohlenstoffkette mit 10 bis 20 Kohlenstoffatomen oder auch aus polyoxalkylierten Alkylamid- oder Alkylethercarboxylsäuren zusammengesetzt ist.

12. Zusammensetzung gemäß Anspruch 10 zum Waschen,
dadurch **gekennzeichnet**, daß
das nicht-ionische oberflächenaktive Co-Mittel aus Alkoholen, α-Diolen oder Alkylphenolen, polyethoxylierten oder polypropoxylerten Fettsäuren mit einer Fettkette mit 8 bis 18 Kohlenstoffatomen, wobei die Anzahl an Ethylen- oder Propylenoxidgruppen 2 bis 50 und die Anzahl an Glycerylgruppen 2 bis 30 betragen, aus Copolymeren aus Ethylen- und Propylenoxiden, Kondensaten von Ethylen- und Propylenoxiden mit Fettalkoholen, polyethoxylierten Fettamiden, polyethoxylierten Fettaminen, Zuckerfettsäureestern, Polyethylenglycolfettsäureestern, Fettsäureestern von Glycolen und aus Aminoxiden ausgewählt ist.

13. Zusammensetzung gemäß Anspruch 10,
dadurch **gekennzeichnet**, daß
das zusätzliche amphotere oder zwitterionische oberflächenaktive Co-Mittel aus sekundären oder tertiären aliphatischen Aminderivaten, in denen der aliphatische Rest eine lineare oder verzweigte Kette mit 8 bis 18 Kohlenstoffatomen ist und mindestens eine anionische, Wasserlöslichkeit vermittelnde Carboxylat-, Sulfonat-, Sulfat-, Phosphat- oder Phosphonatgruppe enthält, aus C₈₋₂₀-Alkylbetainen, Sulfobetainen, C₈₋₂₀-Alkylamido-C₁₋₆-alkylbetainen oder C₈₋₂₀-Alkylamido-C₁₋₆-alkylsulfobetainen, Alkylpeptiden und aus Alkylimidazoliumbetainen ausgewählt ist.

14. Zusammensetzung gemäß Anspruch 10 zum Waschen,
dadurch **gekennzeichnet**, daß
das zusätzliche kationische oberflächenaktive Co-Mittel aus quaternären Ammoniumsalzen ausgewählt ist.

15. Zusammensetzung gemäß einem der Ansprüche 1 bis 14,
dadurch **gekennzeichnet**, daß
sie zusätzlich ein Verdickungsmittel und/oder ein Suspendiermittel für das synthetische Kohlenwasserstofföl enthält.

16. Zusammensetzung gemäß Anspruch 15,
dadurch **gekennzeichnet**, daß
das Verdickungsmittel und/oder Suspendiermittel aus Alkanolamiden von Fettsäuren, Polyacrylsäuren, Cellulosederivaten, Estern von Fettsäure und Polyethylenglycol, aus vernetzten Copolymeren aus Acrylamid und einem Monomer, ausgewählt aus Ammoniumacrylat, 2-Acrylamido-2-methylpropansulfonsäure oder aus Methacryloyloxyethyltrimethylammoniumchlorid, aus Polyetherurethanen und aus vernetzten Copolymeren von Methylvinylether/Maleinsäure ausgewählt sind.

17. Zusammensetzung gemäß Anspruch 15,
dadurch **gekennzeichnet**, daß
das Suspendiermittel für das synthetische Kohlenwasserstofföl aus den folgenden Verbindungen ausgewählt ist:
a) aus denjenigen der Formel:
R₃X (II)
worin R₃ ein aliphatischer Rest mit langer Kohlenstoffkette, die gegebenenfalls durch ein oder mehrere Sauerstoffatome unterbrochen ist, und X ein Carboxyl-, Schwefel- oder Phosphorsäurerest oder ein von einer Carboxylsäure oder einem Amid abgeleiteter Rest sind;
b) aus Aminoxiden der Formel: 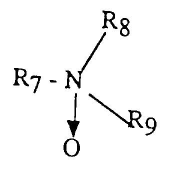 worin R₇ einen C₁₆₋₂₂-Alkylrest und R₈ und R₉, gleich oder verschieden, einen C₁₋₄-Alkyl- oder einen C₁₋₄-Hydroxyalkylrest darstellen;
c) aus Biopolysacchariden.

18. Zusammensetzung gemäß einem der Ansprüche 1 bis 17,
dadurch **gekennzeichnet**, daß
das kosmetisch geeignete Milieu aus Wasser oder aus einer Mischung aus Wasser und einem kosmetisch geeigneten Lösungsmittel zusammengesetzt ist.

19. Zusammensetzung gemäß einem der Ansprüche 1 bis 18,
dadurch **gekennzeichnet**, daß
sie in Form einer mehr oder weniger verdickten Flüssigkeit, eines Gels, einer Emulsion, einer hydroalkoholischen Lotion, einer Dispersion, eines Seifenstücks oder eines Aerosol-Schaums vorliegt.

20. Zusammensetzung gemäß einem der Ansprüche 1 bis 19,
dadurch **gekennzeichnet**, daß
sie zusätzlich Additive enthält, ausgewählt aus Schaumverstärkungsmitteln, Sequestriermitteln, Elektrolyten, Parfüm-Produkten, Konservierungsmitteln, Fettalkoholen, mineralischen, pflanzlichen oder tierischen Ölen, mineralischen, pflanzlichen, tierischen oder synthetischen Wachsen, Ceramiden, UV-Filterstoffen, Perlmuttglanzmitteln, Abfangmitteln für freie Radikale, Biociden, antibakteriellen Mitteln, Mitteln gegen Haarausfall, antiseborrheischen Mitteln, Mitteln gegen Parasiten, Abschreckmitteln, Farbstoffen, Pigmenten, Oxidations-, Reduktionsmitteln, Hydratisiermitteln, anionischen oder nicht-ionischen Polymeren, Vitaminen oder aus α-Hydroxysäuren.

21. Verwendung der in einem der Ansprüche 1 bis 20 definierten Zusammensetzungen zum Behandeln und/oder Waschen keratinischer Materien aus Haaren oder der Haut.

22. Verfahren zum Waschen und/oder kosmetischen Konditionieren der Haare oder der Haut,
dadurch **gekennzeichnet**, daß
man auf die Haut oder die Haare eine ausreichende Menge einer Zusammensetzung gemäß einem der Ansprüche 1 bis 20 aufbringt, worauf auf diese Aufbringung gegebenenfalls eine Spülung mit Wasser erfolgt.
